# EUROPEAN PATENT APPLICATION

(11) **EP 3 838 116 A1**
(43) Date of publication of application: **23.06.2021**
(21) Application number: 19218613.8
(22) Date of filing: 20.12.2019
(51) Int. Cl.: A61B 5/00, A61B 5/107, G06T 7/50, A61B 5/11

(54) **SYSTEM FOR DETECTING A CHANGE IN CONTOUR OF A LIVING TISSUE**

(71) Applicant: Nagravision S.A., 1033 Cheseaux-sur-Lausanne (CH)
(72) Inventor: Villegas, Karine, 1033 Cheseaux-sur -Lausanne (CH); Villegas, Manuel, 1033 Cheseaux-sur -Lausanne (CH)
(74) Representative: Hoyng Rokh Monegier B.V.

(57) **Abstract**

Monitoring and detecting changes in contour of the portion of living tissue by comparing a reference model with data representative of one or more parameters of contour of a portion of a living tissue, and detecting if a change in one or more parameters of contour exceeds or is within a threshold value set by the reference model, wherein the threshold value is generated based on a patient-specific parameter and at lest one parameter of the contour of the portion of the living tissue.

## Description

### FIELD OF THE INVENTION

The present disclosure generally relates to methods and systems for use in monitoring and detecting changes in contour of the portion of the living tissue, more particularly to methods and systems for use in monitoring and detecting edema.

### BACKGROUND

Elevated or abnormal fluid levels in living tissue can result from a number of physiological conditions. For example, edema is an abnormal accumulation of fluid in the intercellular spaces of connective tissue. Edematous tissues are swollen and when punctured secrete an incoagulable fluid. Edema is a symptom of disease and caused generally due to alteration in the physiological mechanisms that maintain a constant water balance in the cells, tissues, and blood. Among the causes may be diseases of the kidneys, veins, or lymphatic system; surgery; or chemotherapy. Because dependent edema is a physical indication of a number of different disease conditions, the development of accurate methods for the detection of edema is of interest.

Edema may be detected by visits to medical-care providers, such as physicians, nurses and massage therapists. However, frequent visits to medical-care providers are inconvenient to the patient.

It is possible to place various electronic devices on a patient to detect or monitor edema condition. For example, pressure sensors may be attached to the patient's limb by bandages to monitor the edema condition; if swelling occurs, the pressure sensors will detect the corresponding changes to the pressure exerted by elasto-compression. The pressure sensors are wired to an electronic device that may receive and analyze the signals produced by the pressure sensors.

The existing methods require patients to be physically connected to electrical devices. For examples, wires are used to connect sensors that are normally placed on patients' skin to an electronic device. This significantly disturbs the patient's mobility. In addition, detection accuracy may be reduced due to pressure change excreted on the sensors, for example, when the bandage becomes loose or the sensors become displaced.

In view of the serious health issues caused by abnormal accumulation of fluid in living tissue, and the extreme importance of its early detection, it would be desirable to provide devices, systems and methods for use in monitoring and/or detecting changes in contour of the portion of a living tissue.

It is desirable to develop more accurate devices, systems and methods for detecting changes in contour of a portion of the living tissue, and particularly, for monitoring and/or detecting abnormal shape of the portion of the living tissue, for example, as a result of edema.

It would also be desirable to provide methods and systems for use in monitoring and/or detecting changes in contour of a portion of a living tissue that reduce the need for frequent visits to medical-care providers, are easy to use, that minimizes or avoids wiring electronic devices to the patient.

The present disclosure provides such devices, systems and methods that solve one or more of the problems mentioned above. Other features and advantages of the disclosure will be apparent from the following description and from the claims.

### BRIEF DESCRIPTION OF THE DRAWINGS

The features and advantages of the disclosure will be appreciated upon reference to the following drawing, in which:
Fig. 1 illustrates an exemplary environment in which a method and an image-capturing device in accordance with an embodiment of the present disclosure may be applied.
Fig. 2 illustrates two exemplary images acquired by an exemplary image-capturing device in accordance with an embodiment of the present disclosure.
Fig. 3 illustrates an exemplary image-capturing device in accordance with an embodiment of the present disclosure.
Fig. 4 shows a hardware infrastructure for implementing an embodiment.

The figure is meant for illustrative purposes only, and does not serve as restriction of the scope or the protection as laid down by the claims.

### DESCRIPTION OF ILLUSTRATIVE EMBODIMENTS

The devices, computer programs, systems, and methods of the present disclosure are applicable to the monitoring any abnormal contour of a portion of a living tissue caused, for example, by fluid accumulation within the living tissue (whether a body fluid or an introduced fluid). Detection of contour of a portion of living tissue according to the present disclosure includes, but is not limited to, the detection of change in contour and shape of the portion of the living tissue as a result of edema, ruptured organ and the like.

The term "contour" has a well-accepted meaning in the field, for instance, a "contour", for the case of a black-and-white image of the portion of the living tissue, is a closed enveloping line encompassing closed areas of the same color (black or white) of the portion of the living tissue. The term "contour, as used herein also refers to the outline of an anatomical feature of the living tissue, typically represented by the edge or line that defines or bounds the shape of the anatomical feature of the portion of the living tissue. The term "contour" as used herein includes shape of a portion of the living tissue that segments the portion boundaries. Those of ordinary skill in the art are aware of various methods and devices (e.g. sensors) used for detecting contour of an object and the said properties.

The term "contour" of a portion of the living tissue includes "shape" of the portion of the living tissue. For example, detecting and/or monitoring contour of a portion of a living tissue can include detecting and/or monitoring shape of a portion of a living tissue. The term "shape", as used herein, relates to the two-dimensional or three-dimensional shape of a portion of a living tissue image, in the field of view. A portion of the living tissue's shape is considered to have various degree of regularity or irregularity. The criterion to determine the shape of portion of the living tissue is by employing properties like perimeter, bounding box, convex, concave, axis, centroid, volume, pressure, and electrical conductance. Those of ordinary skill in the art are aware of various methods and devices (e.g. sensors) used for detecting shape of an object and the said properties.

In one aspect of the present disclosure, a method for monitoring and/or detecting changes in contour of a portion of the living tissue is provided.

In one embodiment, the method comprises processing data set, by a processor, obtained from a sensing unit to extract one or more parameters representative contour of the portion of the living tissue; comparing, by a processor, the extracted one or more parameters with a reference model; determining, by a processor, whether the one or more parameters exceeds or is within a predetermined threshold value set by the reference model based on the comparison; and generating, by a processor, an output comprising an indication of change in contour of the portion of the living tissue, when the one or more parameters exceeds the threshold value.

The sensing unit of present disclosure comprises a sensor and a transmitting device configured to transmit at least data representative of contour of the portion of the living tissue. Optionally, the sensing unit comprises a data receiving device (e.g. transceiver), a processor operatively coupled to the data receiving device (e.g. transceiver) and/or a non-transitory computer readable storage medium connected to the processor.

In one embodiment, the sensing unit of present disclosure is configured to generate data representative of the contour of the portion of the living tissue continuously, optionally, every second, every minute, every day, every month, and/ or months.

Optionally, the sensing unit is in contact with the portion of the living tissue (e.g. sensing unit is placed on the skin of a patient). Optionally, the sensing unit is not in direct contact with a portion of living tissue (e.g. sensing unit is placed on a bandage placed on the portion of the living tissue). Optionally, the sensing unit can be placed in various locations as long as it is capable of detecting and extracting one or more parameters representative contour of the portion of the living tissue.

The one or more parameters representative contour of the portion of the living tissue are detectable by various methods and devices, for example, by various sensors (optionally, the sensors of the sensing unit) including pressure sensor, electroconductivity sensor, by an image processing and analysis software or image-capturing device. For example, the one or more parameters representative contour of the portion of the living tissue comprises at least one from the group consisting of shape, volume, stretch, circumference, 2D image, 3D image, and electrical conductivity of the portion of the living tissue.

The term "patient" refers to any individual (human or animal) with respect to whom the devices, computer programs, methods, and systems of present disclosure are intended to be, or has been, used. Optionally, the patient is human.

The term "living tissue" used herein refers to a biological tissue (also including an organ) which is not fixed or frozen at the time of performing method of present disclosure.

As used herein the term "reference model" refers to predetermined and desired model, sub-model, values, standards, threshold values, or parameters available to a service provider such as medical professionals. For example, certain threshold values are set for each parameter (e.g. swelling or volume of the portion of the living tissue) by medical specialists in the field of edema for the specific patient and/or specific portion of the living tissue in the reference model. The reference model can be used here to model the (e.g. via algorithm) behavior of the portion of the living tissue for the purpose of model-based monitoring for use in detecting and/or monitoring a change in the contour of the portion of the living tissue. The reference model is designed specifically for a particular portion of the living tissue (e.g. living tissues of breast, leg, foot, brain, etc.) and optionally for a particular patient.

In one embodiment, the reference model comprises a predetermined shape of the portion of the living tissue. Optionally, the predetermined shape is associated with the condition of no swelling, thereby allowing detection of any swelling. The predetermined shape of the portion of the living tissue may also be associated with a certain amount of swelling; in this case, changes to the shape of the portion of the living tissue may indicate a deterioration of edema (further swelling) or an improvement. If the improvement is caused by administration of medication, the efficacy of the medication and the recovery speed may also be monitored.

In one embodiment, the reference model of present disclosure in addition to comprising predetermined values for the one or more parameters representative of contour of the portion of the living tissue, it also comprises at least one patient-specific's parameter.

As used herein the term "patient-specific parameter" refers to a variety of information which is unique to each patient. For example, the patient-specific information includes family medical history information, medical conditions (e.g. allergy information, diseases,), exercise information, drug prescriptions, skin and body conditions (e.g. skin type, skin texture, muscle tone, etc.), age of the patient or any combinations of any of the patient-specific parameter.

The patient-specific parameters according to present disclosure can also include imaging data collected from the patient, for example, imaging data from one or more of x-ray imaging, MRI, PET, ultrasound, and is used to qualitatively and/or quantitatively measure one or more of a patient's biological features. The measured biological features can include shape, for example, two-dimensional shape or three-dimensional shape; area, for example, surface area and/or surface contour; perimeter shape; and/or volume of, for example, the patient's cartilage, bone.

In addition to (or optionally in place of) the above-mentioned measurements, it may be desirable to have the reference model to include measurements (one or more parameters) of the contour of the portion of living tissue as well as surrounding anatomical areas of the patient's anatomy. Such measurements can provide, for example, data on movement of the portion of the living tissue, which can be more prone to change in its contour.

Optionally, the reference model is generated (e.g. its threshold value has been determined) based on combination of the one or more parameters of the contour of the living tissue and patient-specific parameter. Optionally, in the reference model, the one or more parameters of the contour of the portion of the living tissue are normalized with a patient-specific information.

In one embodiment, the predetermined reference model is modified according to the one or more parameters of the portion of the living tissue and, optionally, the patient-specific parameter. The reference model can be modified before or during operation of the sensing unit, for example, before or during performing method(s) of present disclosure.

Optionally, the reference model is stored in at least one sensing unit, remote device (e.g. server, smartphone or camera) or a storage medium such as non-transitory computer readable storage medium according to present disclosure.

In an embodiment, the method of present disclosure comprises generating, by a processor, a signal in response to a result of the comparison of the extracted and measured one or more parameters of the contour of the portion of the living tissue and the reference model. For example, if the result of the comparison indicates that the contour (e.g. volume, shape, edge line, etc.) of the portion of the living tissue that is being monitored exceeds a threshold level as set in a reference model, a signal in the form of an alarm may be generated. The signal could attract attention of the patient and/or the medical-care provider. A signal may also be generated when the comparison indicates that the physiological condition (e.g. edema) being monitored has been stable.

In an embodiment, the signal is a visual, audio, or tactile alert. A visual signal may be in the form of a warning message on a screen of an electronic device of the patient or the medical-care provider. A visual signal may also be in the form of blinking lights emitted from, e.g., an LED on the patient's smartphone. An audio or tactile signal may be useful in situations such as when the patient or the medical-care provider has fallen asleep but urgent medication attention is required.

In an embodiment, the extracting and measuring one or more parameters of the contour of the portion of the living tissue, and the comparing of the measured and extracted one or more parameters with the reference model are each performed a plurality of times, optionally, continuously, twenty four hours a day. Repeated measurements and analysis may increase the monitoring accuracy. The measurements and analysis may be averaged out to allow the trend to show or be filtered to remove the effects of short-term disturbances.

In an embodiment, the frequency at which the acquiring, the measuring and the comparing is performed depends at least in part on one or more of the previously measured one or more parameters (e.g. shapes).This may allow optimization of the attention needed from the patient and/or the medical-care provider, giving them more convenience. For example, if prior measurements indicate a stabilizing condition, the patient may adjust its preference to receive fewer signal per day.

In an embodiment, the acquiring, the measuring and the comparing are performed at regular periods; or wherein the acquiring, the measuring and the comparing are performed on-demand.

Performing such actions at regular periods, instead of once, is often useful in effectively monitoring the physiological condition such as edema. The periods may be measured in seconds, minutes, hours or days. Alternatively or additionally, the acquiring, the measuring and the comparing may be performed on-demand based on, e.g., the patient's feeling of his own health, the medical-care provider's instruction, or the recommendation from software that analyzes the patient's historical data.

In another aspect of the present disclosure, a non-transitory computer readable storage medium is provided.

The term "non-transitory computer-readable medium" as used herein refers to any medium that comprises the actual performance of an operation (such as hardware circuits), that comprises programs and/or instructions to be provided to one or more processors for performance/implementation (such as instructions stored in a non-transitory memory), and/or that comprises instructions stored in memory. Non-transitory computer-readable media may take many forms, such as nonvolatile and volatile media, including but not limited to, a floppy disk, flexible disk, hard disk, RAM, any memory chip, any magnetic medium from which a computer instruction can be read; a CD-ROM, DVD, or any other optical medium from which a computer instruction can be read, or any other non-transitory medium from which a computer instruction can be read.

In one embodiment, the programs and/or higher-level instructions in the non-transitory computer-readable medium intend to be used for one or more medical purposes such as detecting or monitoring edema. Optionally, the programs and/or higher-level instructions in the non-transitory computer-readable medium intend to be used without being part of a hardware medical device. "without being part of' means software not necessary for a hardware medical device to achieve its intended medical purpose.

In one embodiment, the non-transitory computer readable storage medium is used for detecting and/or monitoring a change in contour of the portion of the living tissue, optionally, for use in detecting and/or monitoring edema. The non-transitory computer readable storage medium is configured to store instructions that when executed cause a processor to perform comparing data representative of one or more parameters of contour extracted from a portion of a living tissue with a reference model.

In one embodiment, the non-transitory computer-readable storage medium according to present disclosure is configured to store instructions that when executed cause a processor to perform comparing data representative of one or more contour parameters extracted from the portion of a living tissue with a reference model; wherein the instruction configured that when executed to cause the processor to detect and/or to monitor whether the extracted one or more parameters of the contour of the portion of the living tissue exceeds and/or is within a threshold value predetermined by the reference model based on the comparison and generating an alert output when the one or more parameters of the contour of the portion of the living tissue exceeds the predetermined threshold value.

When the one or more parameters of the contour of the portion of the living tissue exceeds the predetermined threshold value, this can be indicative that the level of fluid in the living tissue has been changed or it is changing.

Optionally, the non-transitory computer-readable storage medium according to any of the paragraphs is configured to store instructions that when executed cause a processor to receive data representative of the reference model, which is, for example, predetermined based on one or more parameters of the contour of the specific portion of the living tissue, alternatively or additionally, the reference model can be predetermined based at least on one patient-specific parameter such as parameters of the specific patient being monitored (patient-specific parameter).

The non-transitory computer-readable storage medium of any of the paragraphs, wherein the non-transitory computer-readable storage medium configured to store instructions that when executed cause a processor to compare at least one extracted parameter during a first period of time with a reference model and to compare at least one extracted parameter during a second period of time with a reference model; generating an output indicative progression of change in one or more parameters of the contour of the portion of the living tissue. The output can be indicative whether level of fluid in the living tissue has been increased, maintained, or reduced.

One skilled in the art will appreciate that any of paragraphs of the present disclosure such as paragraphs to the reference model and the one or more parameters can be applied and it is incorporated by reference in its entirety to paragraphs of the non-transitory computer-readable storage medium.

In another aspect of present disclosure, a device comprising a processor and, optionally, a non-transitory computer-readable storage medium according to any of the paragraphs is provided. The device of present disclosure is for use in detecting and/or monitoring a change in contour of the portion of the living tissue, optionally for use in detecting and/or monitoring edema.

The device of the present disclosure is located outside the patient and it is configured to perform at least one from a the following functions: to take a photographic image of the portion of the living tissue; to extract one or more parameters representative of contour of the portion of the living tissue; to store a reference model; to store and update the reference model when needed; to compare the extracted one or more parameters of the contour of the portion of the living tissue with a reference model; to detect and/or monitor change in one or more parameters of the contour of the portion of the living tissue; to produce a measurable output; to produce an output indicative a change in contour of the portion of the living tissue; and to produce an output indicative of a change in a level of fluid in living tissue when the extracted one or more parameters exceeds the predetermined threshold values as set in the reference model.

The device of the present disclosure is optionally located outside the patient. The device of the present disclosure is designed to detect, read, monitor, or simply is responsive to one or more of the sensing units of the present disclosure and to produce a measurable output. For example, the device is a server. Optionally, at least one device is being designed to wirelessly power the sensing units. Optionally, at least one device of the present invention is designed to be any electrical filed emitting, electromagnetic wave-emitting, electromagnetic wave-detecting, and/or electromagnetic wave-activated and activating device, such as, for example, radio frequency identification (RFID) transponders or tags, microwave tags, inlays, etc. More than one device can be used to transmit and to receive signals to and from different sensing units placed at different locations.

Optionally, the output of the image-capturing device may be measured and displayed by various types of consumer electronics devices, including, but not limited to, personal computers, digital video disk devices, television sets, audio reproduction systems, smart phones, smart watches, and notebooks. However, in various alternate embodiments, any appropriate electronic network designed to permit communication between any desired types of electronic devices.

In an embodiment, the device comprises a wireless communication module, a secure communication module, or both. The wireless communication module may make it easy for the patient to communicate the received images and/or analysis data from the sensing unit with, e.g., a physician or a nurse. Since the data may contain sensitive personal and/or medical information, a secure communication module could strengthen the patient's privacy.

In an embodiment, the secure communication module ensures confidentiality and/or integrity and/or authenticity of the data transmitted from or received by the image-capturing device. Data confidentiality protects the patient's privacy. Integrity ensures that the data in transit are not corrupted or tempered with. Authenticity ensures, e.g., the data received by the physician indeed come from the correct patient.

In an embodiment, the processor comprises a computation module, preferably a CPU; wherein the computation module compares the measured and extracted one or more parameters of the contour of the portion of the living tissue with a predetermined reference model. In one embodiment, the computation model is coupled with the non-transitory computer readable storage medium of present disclosure.

In one embodiment, the device comprises a computation module. With such computation module, the device can make the comparison itself without relying on another device (e.g. a server, a computer). For example, a smartphone may measure and extract one or more parameters representative of contour of the portion of the living tissue and perform the processing at the same time to provide instant feedback to the patient. The smartphone may also communicate the feedback to a remote device, such as a physician's computer.

In an embodiment, the computation module is configured to receive algorithmic training from a remote server, the algorithmic training relating to the measuring the one or more parameters of the contour of the portion of the living tissue (e.g. shape, volume, edge line, etc.) and/or the comparing of the measured one or more parameters with the reference model.

The computation module enables the device to process data locally, instead of having to rely on a remote server. However, a remote server can also be used to perform measurement and comparison according to the present disclosure. For example, the computing power of the image-capturing device may be limited because of its size or portability. This limitation may make it less feasible to implement some algorithms or algorithmic training that improve the performance of data processing but often require large computational power. The remote server that provides algorithmic training to the device may obviate this limitation.

It is also possible that the computation module of the device itself possesses sufficient computational power to locally train the processing algorithms, such as shape measurement and comparison.

In an embodiment, the device pushes at least one of its functions to another remote device such as server or smartphone of medical practitioner.

Pushing is a form of proactively communicating data from the device to another device. This may reduce delay in situations where further actions by the patient or the physician depend on information reaching said another device. Pushing the result of the comparison may create an early alert to the physician or nurse remotely monitoring the patient.

In an embodiment, the computational module detects a trend from the comparison between the extracted one or more parameters representative contour of the portion of the living tissue in a plurality of acquired images with the reference model. This allows continuous monitoring of the target living tissue and fluid level. It also enables monitoring progress of the medication that treats the target living tissue such as edema.

In another aspect of present disclosure, a server device is provided. The server is for use in detecting and/or monitoring a change in contour of the portion of a living tissue, optionally for use in detecting and/or monitoring edema. The server device comprises a data receiving device such as a transceiver configured to receive data representative of the extracted one or more parameters representative of contour of the portion of the living tissue generated during a time period by the sensing unit or the device of present disclosure; a processor operatively coupled to the transceiver; and one or more non-transitory computer readable storage medium according to any of the paragraphs operatively connected to the processor.

In this embodiment, it is clear that any of the paragraphs of the present disclosure is incorporated by reference to the embodiments representing the server. For example, all the paragraphs of present disclosure related to a non-transitory computer readable storage medium configured to store instructions that when executed cause a processor to perform any functions mentioned above alone or in combination; the image capturing device are incorporated by reference.

According to an aspect of the present invention, there is provided a system for use in detecting and/or monitoring a change in contour of a portion of a living tissue, optionally for use in detecting and/or monitoring edema, said system comprises a device (e.g. remote element such as camera, smartphone or a computer) configured to receive the output from the sensing unit, the non-transitory computer-readable medium, the server configured to receive data from the device, optionally, the server is configured to receive data from the sensing unit.

In one embodiment, the system comprises: a server configured to receive data representative of one or more parameters extracted from the contour of the portion of the living tissue sent from the device and/or the sensing unit of present disclosure; a processor, the processor operable for: receiving the data set from the device or the sensing unit; processing the data set to extract one or more parameters representative contour of the portion of the living tissue; comparing the extracted one or more parameters with a reference model and generating an output comprising an indication of change in the contour of the portion of the living tissue. Optionally, the processor is configured to be coupled to the non-transitory computer-readable storage medium according to any of the paragraphs of present disclosure.

The non-transitory computer readable storage medium; servers; systems; devices; and methods according to any of the paragraphs of present disclosure address the problem of fluid accumulation in a living tissue. They capture change in contour of the portion of the living tissue as a result of change in fluid level of that portion of living tissue outside of the clinical settings and enable the patient engagement. Unlike current procedures of connecting electrical devices with wires to patients and continuous monitoring by professional healthcare providers, the present disclosure enables patients to monitor and to detect edema at very early stage by monitoring the contour of their living tissue as frequent as needed without a need to visit professional every time.

The non-transitory computer readable storage media; servers; systems; medical devices; and methods according to any of the paragraphs of present disclosure are able to monitor/screen/detect a disease or condition (such as edema) via detecting a change in contour of the portion of the living tissue, to identify early signs of a disease or conditions, to inform the patient and medical practitioners about the available options to reduce or to prevent fluid accumulation, and to provide clinical information by aggregating relevant information.

### EXAMPLES

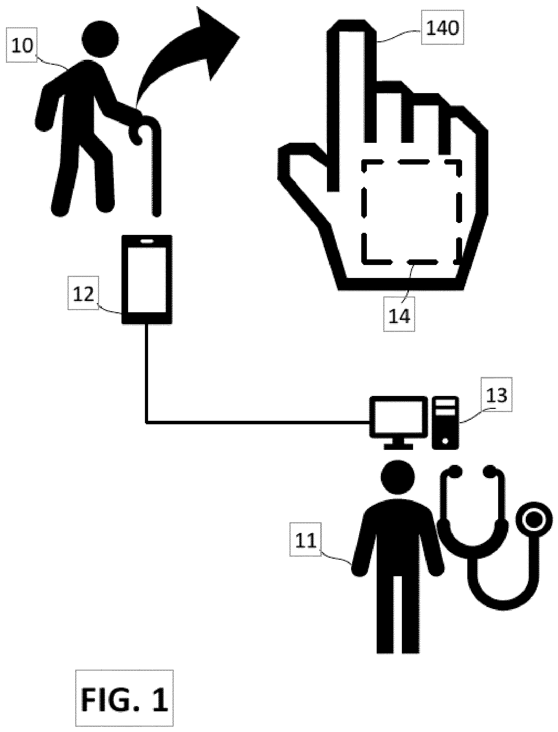

Fig. 1 illustrates an exemplary environment in which a method and an image-capturing device in accordance with an embodiment of the present disclosure may be applied. There is a patient 10 and a medical-care provider 11, such as a physician, who may want to detect and/or monitor change in contour of a portion of a living tissue of the patient 10 and perform treatments if necessary. The patient 10 possesses an image-capturing device 12, such as a mobile phone. The physician 11 may have a processing device 13, such as a desktop, a laptop, a mobile phone or a tablet. The patient 10 may use his image-capturing device 12 to acquire pictures of the living tissue area that is being monitored. In the example illustrated in Fig. 1, the living tissue area that is being monitored is on his right hand 140.

Monitoring and/or detecting change in countour of living tissue of the patient 10 may require regular or periodic monitoring. An example is tissue or limb swelling caused by inflammation or edema, which, if left unattended, often result in undesirable medical consequences.

The physician 11 may inspect the patient 10 to monitor the change in countour in the target living tissue. Requiring the patient 10 to visit the physician's office for every inspection is burdensome.

Instead, the patient 10 may use his image-capturing device 12 to take an image of the target living tissue 14. The image-capturing device 12 may analyze (e.g. extract one or more parameters, optionally, compare them with a reference model) the image 14 and, depending on the analysis result, generate a signal to notify the patient 10 or the physician 11. The image-capturing device 12 may also send the image to the processing device 13 for the physician 11 to review. The processing device 13 may also analyze the image 14 prior to the physician's review and, depending on the analysis result, generate a signal.

The image-capturing device 12 may be a camera or a mobile device comprising a camera, such as a smartphone or a tablet. The image-capturing device 12 may comprise a image acquisition module, a computation module (such as a central processing unit, CPU), a communication module, a memory, a non-transitory computer readable storage medium according to present disclosure.

The image-capturing device 12 is communicatively coupled to the processing device 13. The communicative coupling may be wired or wireless. The communicative coupling may be secure to ensure the confidentiality, integrity and/or authenticity of the data communicated between the image-capturing device 12 and the processing device 13.

The processing device 13 does not have to be associated with the physician 11. For example, the processing device 13 may be a remote server of a third-party provider that offers services such as medical data analysis and management.

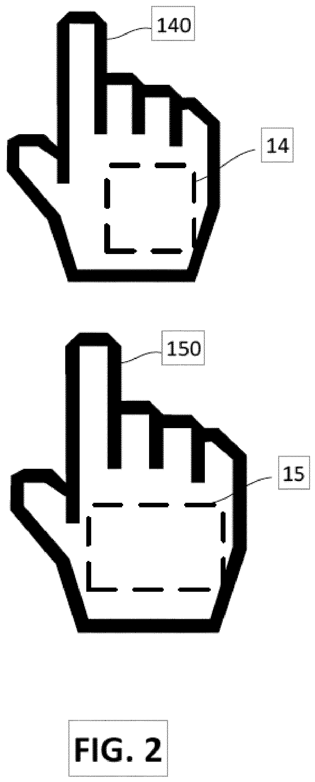

A more detailed explanation of the present disclosure will be made by referring to Fig. 2, which illustrates two exemplary images 14 and 15 acquired by the image-capturing device 12.

The images 14 and 15 are acquired at different times. The image 14 taken from a portion of the living tissue on the hand 140. When the image 15 is taken, there might have been changes to the level of fluid in the tissue, making the hand 150 swell and become bigger than the hand 140 in the image 14.

The contours, e.g. shapes of images 14 and 15 may be measured. The measurement may be performed by the image-capturing device 12 itself; or the images 14 and 15 may be sent to another computer for the measurement. The measured shapes obtained by images 14 and 15 are then compared to each other. Likewise, the comparison may be done by the software in the image-capturing device 12 itself or in another computer communicatively coupled to the image-capturing device 12, such as the processing device 13 illustrated in Fig. 1.

Compared to the shape of the living tissue in image 14, the shape of the living tissue in image 15 may indicate that there has been swelling, which may be caused by a worsening edema condition. Depending on the amount of difference, a signal may be generated to notify the patient 10 or the physician 11. The signal may take the form of a visual, audio, or tactile alert. The visual alert may be in graphical forms (such as an alarm icon on the patient's image-capturing device 12) or in texts (such as an email sent to the physician's email account).

In an embodiment, the difference between the shapes of the images is, for example, quantified. The signal may be generated only if the quantified difference exceeds a threshold generated by the reference model. The threshold may vary according to, for example, patient's specific parameters such as patient's medical condition.

The difference in time between when the images 14 and 15 are taken may be regular, such as every 0.5, 1, 2, 3, 4, 5, 6, 7, 8, 9 or 10 hours. The difference in time between when the images 14 and 15 are taken may depend on the patient's medical history and/or the physician's judgement.

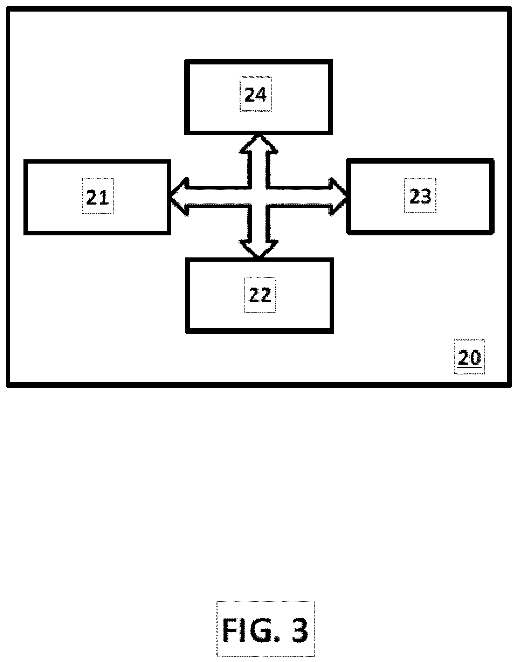

Fig. 3 illustrates an exemplary image-capturing device 20 in accordance with an embodiment of the present disclosure.

The image-processing device 20 comprises an image acquisition module 21, a computation module 22, a communication module 23 and a memory 24. As shown in the cross arrow in Fig. 3, the modules 21, 22, 23 and the memory 24 may be communicatively (e.g., electronically) coupled to each other. The image-processing device 20 may be a camera or a smart mobile device.

The image acquisition module 21 may comprise a lens, light sensors and color sensors. The image acquisition module 21 may be configured to acquire images from the target living tissue to be analyzed. The image acquisition module 21 may be a camera in a smartphone. As is popular nowadays, there may be more than one image acquisition modules 21 in the image-processing device 20.

The computation module 22 may a central processing unit (CPU). The computation module 22 may be configured to process the images acquired by the image acquisition module 21, such as detecting and measuring the contour, e.g. shape of target living tissue in the acquired images and comparing among the measured shapes. The computation module 22 may be configured to process multiple images and detects a trend in the comparison results. The computation module 22 may be configured to receive algorithmic training. The training may be based on pattern recognition training models. The training may be based on artificial-intelligence techniques.

The communication module 23 may be configured to transmit data from the image-processing device 20 and receive data to the image-processing device 20. The communication module 23 may provide wired and/or wireless communication capabilities. The communication module 23 may also provide secured communication capability to protect the confidentiality, integrity and authenticity of the data. There capabilities may be implemented within the same module (e.g., as in different software blocks at the logical layer) or as different sub-modules (e.g., transceiver, modem, encryption/decryption engine).

The communication module 23 may be configured, at the request of the computation module 22 for example, to push various information to a remote processing device. The information that can be pushed includes images acquired by the image acquisition module 21, shapes of various objects in the acquired images measured by the computation module 22, and the result of the comparison by the computation module 22.

The memory 24 may store various information. One information that is useful to store in the memory 24 is the predetermined shape of the artificial pattern. Such predetermined shape may serve as a baseline for some or all of the measurements, comparison and computation performed by the computation module 22.

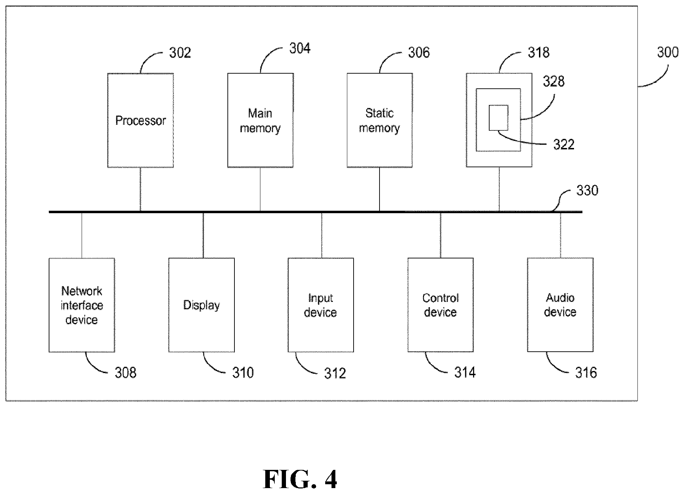

Figure 4 illustrates a block diagram of one implementation of a computing device 300 within which a set of instructions, for causing the computing device to perform any one or more of the methodologies discussed herein, may be executed. The computing device 300 may be used for elements of a system used to carry out the present disclosure.

In alternative implementations, the computing device may be connected (e.g., networked) to other machines in a Local Area Network (LAN), an intranet, an extranet, or the Internet. The computing device may operate in the capacity of a server or a client machine in a client server network environment, or as a peer machine in a peer-to-peer (or distributed) network environment.

The computing device may be a personal computer (PC), a tablet computer, a set-top box (STB), a wearable computing device, a Personal Digital Assistant (PDA), a cellular telephone, a web appliance, a server, a network router, switch or bridge, or any machine capable of executing a set of instructions (sequential or otherwise) that specify actions to be taken by that machine. Further, while only a single computing device is illustrated, the term "computing device" shall also be taken to include any collection of machines (e.g., computers) that individually or jointly execute a set (or multiple sets) of instructions to perform any one or more of the methodologies discussed herein.

The example computing device 300 includes a processing device 302, a main memory 304 (e.g., read-only memory (ROM), flash memory, dynamic random access memory (DRAM) such as synchronous DRAM (SDRAM) or Rambus DRAM (RDRAM), etc.), a static memory 306 (e.g., flash memory, static random access memory (SRAM), etc.), and a secondary memory (e.g., a data storage device 318), which communicate with each other via a bus 330.

Processing device 302 represents one or more general-purpose processors such as a microprocessor, central processing unit, or the like. More particularly, the processing device 302 may be a complex instruction set computing (CISC) microprocessor, reduced instruction set computing (RISC) microprocessor, very long instruction word (VLIW) microprocessor, processor implementing other instruction sets, or processors implementing a combination of instruction sets. Processing device 302 may also be one or more special-purpose processing devices such as an application specific integrated circuit (ASIC), a field programmable gate array (FPGA), a digital signal processor (DSP), network processor, or the like. Processing device 302 is configured to execute the processing logic (instructions 322) for performing the operations and steps discussed herein.

The computing device 300 may further include a network interface device 308. The computing device 300 also may include a video display unit 310 (e.g., a liquid crystal display (LCD) or a cathode ray tube (CRT)), an alphanumeric input device 312 (e.g., a keyboard or touchscreen), a cursor control device 314 (e.g., a mouse or touchscreen), and an audio device 316 (e.g., a speaker).

The data storage device 318 may include one or more machine-readable storage media (or more specifically one or more non-transitory computer-readable storage media 328) on which is stored one or more sets of instructions 322 embodying any one or more of the methodologies or functions described herein. The instructions 322 may also reside, completely or at least partially, within the main memory 304 and/or within the processing device 302 during execution thereof by the computing device 300, the main memory 304 and the processing device 302 also constituting computer-readable storage media.

The various methods described above may be implemented by a computer program. The computer program may include computer code arranged to instruct a computer to perform the functions of one or more of the various methods described above. The computer program and/or the code for performing such methods may be provided to an apparatus, such as a computer, on one or more computer readable media or, more generally, a computer program product. The computer readable media may be transitory or non-transitory. The one or more computer readable media could be, for example, an electronic, magnetic, optical, electromagnetic, infrared, or semiconductor system, or a propagation medium for data transmission, for example for downloading the code over the Internet. Alternatively, the one or more computer readable media could take the form of one or more physical computer readable media such as semiconductor or solid state memory, magnetic tape, a removable computer diskette, a random access memory (RAM), a read-only memory (ROM), a rigid magnetic disc, and an optical disk, such as a CD-ROM, CD-R/W or DVD

In an implementation, the modules, components and other features described herein (for example control unit 310 in relation to Figure 4) can be implemented as discrete components or integrated in the functionality of hardware components such as ASICS, FPGAs, DSPs or similar devices as part of an individualization server.

A "hardware component" is a tangible (e.g., non-transitory) physical component (e.g., a set of one or more processors) capable of performing certain operations and may be configured or arranged in a certain physical manner. A hardware component may include dedicated circuitry or logic that is permanently configured to perform certain operations. A hardware component may be or include a special-purpose processor, such as a field programmable gate array (FPGA) or an ASIC. A hardware component may also include programmable logic or circuitry that is temporarily configured by software to perform certain operations.

Accordingly, the phrase "hardware component" should be understood to encompass a tangible entity that may be physically constructed, permanently configured (e.g., hardwired), or temporarily configured (e.g., programmed) to operate in a certain manner or to perform certain operations described herein.

In addition, the modules and components can be implemented as firmware or functional circuitry within hardware devices. Further, the modules and components can be implemented in any combination of hardware devices and software components, or only in software (e.g., code stored or otherwise embodied in a machine-readable medium or in a transmission medium).

The detailed description set-forth above is provided to aid those skilled in the art in practicing the present invention. However, the invention described and claimed herein is not to be limited in scope by the specific embodiments herein disclosed because these embodiments are intended as illustration of several aspects of the invention. The embodiments set-forth above can be performed and combined with other disclosed embodiments according to the invention. Any equivalent embodiments are intended to be within the scope of this invention. Indeed, various modifications of the invention in addition to those shown and described herein will become apparent to those skilled in the art from the foregoing description which do not depart from the spirit or scope of the present inventive discovery. Such modifications are also intended to fall within the scope of the appended claims. All publications, patents, patent applications and other references cited in this application are incorporated herein by reference in their entirety for all purposes to the same extent as if each individual publication, patent, patent application or other reference was specifically and individually indicated to be incorporated by reference in its entirety for all purposes. Citation of a reference herein shall not be construed as an admission that such is prior art to the present invention.

## Claims

1. A non-transitory computer readable storage medium configured to store instructions that when executed cause a processor to perform:
comparing a reference model with data representative of one or more parameters of contour of a portion of a living tissue generated during a time period by a sensing unit;
detecting and/or to monitoring whether a change in one or more parameters of contour of the portion of the living tissue exceeds and/or is within a predetermined threshold value set by the reference model for the one or more parameters of contour of the portion of the living tissue based on said comparison;
wherein the predetermined threshold value as set by the reference model is generated at least based on a patient-specific parameter and at least one parameter of the contour of the portion of the living tissue.

2. The non-transitory computer readable storage medium according to claim 1, wherein the patient-specific parameter comprises at least one from the group consisting of patient's age, patient's weight, patient's activity level, and patient's medical condition.

3. The non-transitory computer readable storage medium according to any of the preceding claims, wherein the at least one parameter of the contour of the portion of the living tissue comprises at least one from the group consisting of area, perimeter, centroid, bounding box, convex, and concave.

4. The non-transitory computer readable storage medium according to any of the preceding claims, wherein the instructions configured that when executed to cause a processor to perform receiving data representative of one or more parameters of the contour of the portion of the living tissue generated during a time period by the sensing unit.

5. The non-transitory computer readable storage medium according to any of the preceding claims, wherein the instructions configured that when executed to cause a processor to perform sending data representative of one or more parameters of the contour of the living tissue generated during a time period by the sensing unit to a device.

6. The non-transitory computer readable storage medium according to any of the preceding claims, wherein the instruction configured that when executed to cause the processor to display, on a display, an output, wherein said output comprises a notification that the one or more parameters of the contour of the portion of the living tissue has been changed or it is changing based at least in part on the comparison of the one or more parameters of the contour of the portion of the living tissue with the reference model.

7. The non-transitory computer readable storage medium according to any of the preceding claims, wherein the sensing unit is configured to generate data representative of the one or more parameters of the contour of the living tissue every second, every minute, every day, every month, and/ or months; and wherein the instructions configured that when executed to cause a processor to perform comparing the data with the reference model every second, every minute, every day, every month, or months.

8. The non-transitory computer-readable storage medium according to any of the preceding claims, wherein the sensing unit comprises at least one sensor that is configured to detect and to convey at least one from the group consisting of circumference, 2D image, 3D image, volume, of the portion of the living tissue; pressure excreted by the portion of the living tissue; and electrical conductance of the portion of the living tissue.

9. The non-transitory computer-readable storage medium according to any of the preceding claims, wherein the non-transitory computer-readable storage medium configured to store instructions that when executed cause a processor to compare plurality of parameters of contour of the portion of the living tissue with a reference model during a first period of time and to compare plurality of parameters of the contour of the portion of the living tissue with the reference model during a second period of time; generating an output indicative progression of change in at least one parameter of the contour of the living tissue based on comparison with a predetermined threshold value as set by the reference model obtained during the first and second period of time.

10. The non-transitory computer-readable storage medium of any of the preceding claims, wherein the predetermined threshold value of the reference model is determined and adjusted before or during comparing at least one parameter of the contour of the portion of the living tissue with a reference model.

11. A system, comprising:
a server configured to receive data representative of one or more parameters extracted from the contour of the portion of the living tissue sent from a sensing unit;
a processor, the processor operable for:
receiving the data set from the sensing unit;
processing the data set to extract one or more parameters representative contour of the portion of the living tissue;
comparing the extracted one or more parameters with a reference model and generating an output comprising an indication of change in the contour of the portion of the living tissue;
detecting and/or to monitoring whether a change in one or more parameters of contour of the portion of the living tissue exceeds and/or is within a predetermined threshold value set by the reference model for the one or more parameters of contour of the portion of the living tissue based on said comparison;
wherein the predetermined threshold value as set by the reference model is generated at least based on a patient-specific parameter and at least one parameter of the contour of the portion of the living tissue.

12. The system of claim 11, wherein the processor is configured to be coupled to the non-transitory computer-readable storage medium according to any of claims 1-10.

13. A method for monitoring and/or detecting changes in contour of a portion of the living tissue, comprising:
processing the data set to extract one or more parameters representative contour of the portion of the living tissue;
comparing the extracted one or more parameters with a reference model and generating an output comprising an indication of change in the contour of the portion of the living tissue;
detecting and/or to monitoring whether a change in one or more parameters of contour of the portion of the living tissue exceeds and/or is within a predetermined threshold value set by the reference model for the one or more parameters of contour of the portion of the living tissue based on said comparison;
wherein the predetermined threshold value as set by the reference model is generated at least based on a patient-specific parameter and at least one parameter of the contour of the portion of the living tissue.

14. The method of claim 13, wherein detecting a change in one or more parameters of contour of the portion of the living tissue exceeding the predetermined threshold value set by the reference model is indicative of presence of edema.

15. The method of claim 13, wherein the patient-specific parameter comprises at least one from the group consisting of patient's age, patient's weight, patient's activity level, and patient's medical condition; and wherein the at least one parameter of the contour of the portion of the living tissue comprises at least one from the group consisting of area, perimeter, centroid, bounding box, convex, and concave.
